(19) **Europäisches Patentamt / European Patent Office / Office européen des brevets**

(11) **EP 4 593 711 B1**

(12) # EUROPEAN PATENT SPECIFICATION

(45) Date of publication and mention
of the grant of the patent:
**17.12.2025   Bulletin 2025/51**

(21) Application number: **23772879.5**

(22) Date of filing: **20.09.2023**

(51) International Patent Classification (IPC):
**A61B 6/02** *(2006.01)*    **A61B 6/00** *(2024.01)*
**B01D 19/00** *(2006.01)*    **A61B 6/03** *(2006.01)*

(52) Cooperative Patent Classification (CPC):
**A61B 6/02; A61B 6/035; A61B 6/4488;
B01D 19/0031; B01D 19/0052; B01D 19/0078;
B01D 19/0084; B01D 19/0094**

(86) International application number:
**PCT/EP2023/075863**

(87) International publication number:
**WO 2024/068375 (04.04.2024 Gazette 2024/14)**

(54) **COMPUTED TOMOGRAPHY IMAGING SYSTEM WITH A COMPONENT HAVING A FLUID RESERVOIR**

COMPUTERTOMOGRAPHIEBILDGEBUNGSSYSTEM MIT EINER KOMPONENTE MIT EINEM FLÜSSIGKEITSRESERVOIR

SYSTÈME D'IMAGERIE PAR TOMODENSITOMÉTRIE AVEC UN COMPOSANT AYANT UN RÉSERVOIR DE FLUIDE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **27.09.2022   EP 22198084**

(43) Date of publication of application:
**06.08.2025   Bulletin 2025/32**

(73) Proprietor: **Koninklijke Philips N.V.
5656 AG Eindhoven (NL)**

(72) Inventors:
- **WEISS, Steffen**
  **5656 AG Eindhoven (NL)**
- **RIBBING, Carolina Maria**
  **5656 AG Eindhoven (NL)**

- **DOUGLAS, Alexander Ulrich**
  **5656 AG Eindhoven (NL)**
- **FORTHMANN, Peter**
  **5656 AG Eindhoven (NL)**
- **LIPS, Oliver**
  **5656AG Eindhoven (NL)**
- **VOGTMEIER, Gereon**
  **5656AG Eindhoven (NL)**

(74) Representative: **Philips Intellectual Property &
Standards
High Tech Campus 52
5656 AG Eindhoven (NL)**

(56) References cited:
**CN-A- 112 403 038      JP-A- 2020 113 447
US-A1- 2011 243 296**

**Description**

FIELD OF THE INVENTION

The present disclosure relates to a computed tomography, CT, imaging system that has a component with a fluid reservoir.

BACKGROUND OF THE INVENTION

A CT imaging system has a rotatable gantry. Various components, including for example an X-ray tube, a high-voltage generator for the X-ray tube, and an X-ray detector, may be coupled to the gantry. In-use, the gantry is rotated around an axis of rotation, and the X-ray tube and the X-ray detector are used to generate X-ray attenuation data from different orientations with respect to an object. The X-ray attenuation data is then reconstructed to provide a volumetric image of the object.

Some of the components that are coupled to the gantry of the CT imaging system include a reservoir that contains a fluid. The fluid may be used to provide cooling, or to provide electrical isolation in the component. For instance, an X-ray tube may include such a reservoir. In this case, the fluid contained in the reservoir may be used to remove excess heat that is produced during the generation of X-ray radiation by the X-ray tube. A high-voltage generator may also include such a reservoir. In this case, the fluid contained in the reservoir may be used to provide cooling and/or to provide electrical isolation in the generator.

During their manufacture, the reservoirs of such components are typically filled with fluid to an appropriate level. The reservoir may be sealed in order to prevent fluid from escaping. However, over time, bubbles, or particles may appear in the fluid in the reservoir. For instance, bubbles may be generated in the fluid as a consequence of excess heating of the fluid by the component, or as a consequence of the movement of the component via the gantry, or as a consequence of circulation of the fluid within a pumped cooling circuit. Particles may also appear in the fluid as a consequence of excess heating, or as a consequence of mechanical wear. The bubbles, or particles, can degrade the ability of the fluid to perform its intended purpose. For instance, bubbles in a cooling fluid reduce its thermal conductivity, thereby degrading its ability to conduct heat. Bubbles in an electrical isolation fluid reduce its electrical breakdown voltage, thereby degrading its performance as an electrical isolator. Particles in a fluid can also reduce its electrical breakdown voltage, thereby degrading its performance as an electrical isolator. Consequently, over time, the fluid in such components may need to be replaced so that its degradation does not impact the performance of the component.

A document US 2011/0243296 A1 discloses a computed tomography system that has a gantry with a rotor side that can be rotated around a system axis during operation, at which at least one X-ray tube is mounted. To cool the at least one X-ray tube a liquid cooling system is equipped with a fluid volume filled with cooling liquid, the fluid volume extends over distances of different sizes from the system axis. The fluid volume is located on the rotor of the gantry and thus is exposed to centrifugal force during operation. To increase pressure in the cooling system, a flexible compensation volume and a movable mass element that rotate with the gantry are provided. The mass element is arranged such that the centrifugal force acting on the mass element during operation causes pressure to be exerted on the cooling liquid. CN112403038A may be considered to disclose the subject-matter according the preamble of the claims 1 & 14.

However, there remains a need for improvements in components that are used in CT imaging systems in order to reduce the impact of bubbles and/or particles in their fluidic reservoirs.

SUMMARY OF THE INVENTION

According to one aspect of the present disclosure, a CT imaging system is provided. The CT imaging system includes a rotatable gantry and a component. The component is mechanically coupled to the gantry, and the gantry is configured to rotate the component around an axis of rotation. The component comprises a reservoir for containing a fluid, and a chamber. The chamber is fluidically coupled to the reservoir, and the chamber is configured to receive bubbles or particles in the fluid which are forced radially with respect to the axis of rotation of the gantry as a result of centrifugal forces acting on the fluid during the rotation of the component around the axis of rotation.

In the above system, when the gantry rotates the component around the axis of rotation, bubbles or particles in the fluid in the reservoir, are received in the chamber as a result of centrifugal forces acting on the fluid. By removing the bubbles or particles from the fluid in this manner, the impact of the bubbles or particles on the performance of the component, is reduced.

Further aspects, features, and advantages of the present disclosure will become apparent from the following description of examples, which is made with reference to the accompanying drawings.

BRIEF DESCRIPTION OF THE DRAWINGS

Fig. 1 is a schematic diagram illustrating an example of a CT imaging system 100 including a rotatable gantry 110 and a component 120, in accordance with some aspects of the present disclosure.

Fig. 2 is a schematic diagram illustrating an example of a component 120 that includes a reservoir 140, and a chamber 150 configured to receive bubbles 160, in accordance with some aspects of the present disclosure.

Fig. 3 is a schematic diagram illustrating an example of a component 120 that includes a reservoir 140, and a chamber 150 configured to receive particles 170, in accordance with some aspects of the present disclosure.

Fig. 4 is a schematic diagram illustrating an example of a component 120 that includes a reservoir 140, and a chamber 150 with an elongate shape, in accordance with some aspects of the present disclosure.

Fig. 5 is a schematic diagram illustrating an example of a component 120 that includes a reservoir 140, and a chamber 150 with an elongate shape that extends along a curved path in an opposing direction 210 to a direction of rotation 220 of the gantry, in accordance with some aspects of the present disclosure.

Fig. 6 is a schematic diagram illustrating an example of a component 120 that includes a reservoir 140, and a chamber 150 with a sensor $230_S$, $230_D$, in accordance with some aspects of the present disclosure.

Fig. 7 is a schematic diagram illustrating an example of a parking position for a gantry 110 wherein an opening 190 disposed at the radially innermost position of the reservoir 150 is arranged uppermost with respect to the reservoir, in accordance with some aspects of the present disclosure.

Fig. 8 is a schematic diagram illustrating an example of a parking position for a gantry 110 wherein an opening 190 disposed at the radially outermost position of the reservoir 150 is arranged lowermost with respect to the reservoir, in accordance with some aspects of the present disclosure.

Fig. 9 is a schematic diagram illustrating an example of a CT imaging system 100 including one or more processors 240 and a remote processing device 250, in accordance with some aspects of the present disclosure.

Fig. 10 is a flowchart illustrating an example of a computer-implemented method of generating a service work order request, in accordance with some aspects of the present disclosure.

DETAILED DESCRIPTION OF EMBODIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a CT imaging system, may be implemented in a component for a CT imaging system, in a corresponding manner.

In the following description, reference is made to examples of a component that is coupled to the gantry of a CT imaging system. The component includes a reservoir for containing a fluid. In some examples, the component is an X-ray tube. However, it is to be appreciated that the X-ray tube serves only as an example, and that the component may alternatively be a different component to an X-ray tube. For instance, the component may alternatively be a high-voltage generator, also referred-to as a generator, that is used to deliver electrical energy to the X-ray tube. By way of another example, the component may be an X-ray detector. The component may alternatively be a different component to the example X-ray tube, generator, and X-ray detector.

In some examples, the purpose of the fluid in the reservoirs described herein is to cool a portion of a component. For instance, the fluid in the reservoir may be used to cool a portion of the X-ray tube, or to cool a portion of the X-ray detector. In these examples, the fluid may be referred-to as a cooling fluid. However, it is to be appreciated that the fluid may alternatively serve a different purpose, or an additional purpose, to cooling. For instance, the fluid may provide electrical isolation to a portion of a component. By way of an example, the fluid may be used to provide electrical isolation to a portion of a generator. In these examples, the fluid may be referred-to as an electrical isolation fluid.

In general, the fluid that is contained in the reservoirs in the examples described herein may be any type of fluid that is suitable for its purpose. By way of some examples, fluids that are suitable for cooling may include water, or oil. Fluids that are suitable for providing electrical isolation may include oil. It is noted that the water, or oil, may also include various additives in order to improve its performance.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray™ and DVD. As mentioned above, there remains a need for improvements in components that are used in CT imaging systems in order to reduce the impact of bubbles and/or particles in their fluidic reservoirs.

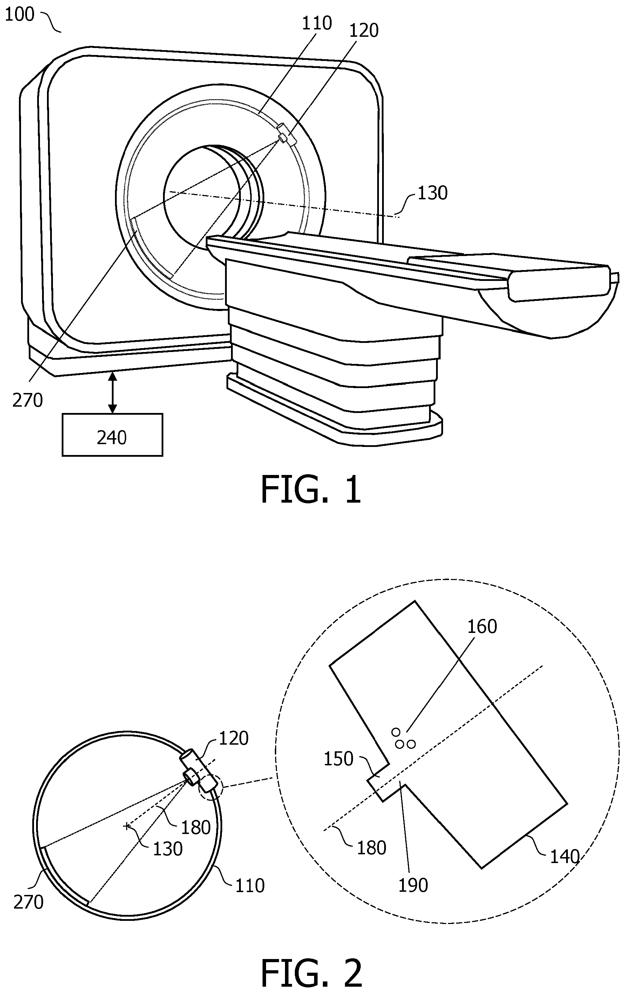

Fig. 1 is a schematic diagram illustrating an example of a CT imaging system 100 including a rotatable gantry 110 and a component 120, in accordance with some aspects of the present disclosure. The rotatable gantry 110 is configured to support an X-ray tube 120 and an X-ray detector 270, as illustrated in Fig. 1. The CT imaging system 100 may be used to perform an imaging operation on an object. For instance, a subject may be positioned on the bed illustrated in Fig. 1 whilst the rotatable gantry 110 is rotated around an axis of rotation 130 in order to generate X-ray attenuation data from different orientations with respect to the subject using an X-ray tube 120 and an X-ray detector 270. The rotatable gantry 110 may be rotated around the axis of rotation 130 using one or more motors or actuators (not illustrated in Fig. 1). The X-ray attenuation data may then be reconstructed to provide a volumetric image of the subject.

With reference to Fig. 1 a CT imaging system 100 is provided that includes a rotatable gantry 110, and a component 120. The component 120 is mechanically coupled to the gantry 110, and the gantry is configured to rotate the component around an axis of rotation 130. The component comprises a reservoir 140 for containing a fluid, and a chamber 150. The chamber 150 is fluidically coupled to the reservoir 140. The chamber is configured to receive bubbles 160 or particles 170 in the fluid which are forced radially 180 with respect to the axis of rotation 130 of the gantry 110 as a result of centrifugal forces acting on the fluid during the rotation of the component around the axis of rotation.

The component 120 illustrated in Fig. 1 is shown in more detail in Fig. 2, which is a schematic diagram illustrating an example of a component 120 that includes a reservoir 140, and a chamber 150 configured to receive bubbles 160, in accordance with some aspects of the present disclosure. The component 120 illustrated in Fig. 2 is mechanically coupled to the rotatable gantry 110 of a CT imaging system. The component 120 illustrated in Fig. 2 may be coupled to the rotatable gantry 110 of the CT imaging system 100 illustrated in Fig. 1, as illustrated by the diagram in the left-hand portion of Fig. 1, and which includes a corresponding rotatable gantry 110 and component 120.

In the example illustrated in Fig. 2, the component 120 is an X-ray tube. In this example, the reservoir 140 contains a fluid for cooling the X-ray tube. When the gantry rotates the X-ray tube around the axis of rotation 130, bubbles 160 in the fluid in the reservoir 140, are received in the chamber as a result of centrifugal forces acting on the fluid.

In the example illustrated in Fig. 2, the chamber 150 is fluidically coupled to the reservoir 140 via an opening 190 in the reservoir. The opening 190 is disposed at a radially innermost position of the reservoir 140 for receiving bubbles 160 in the fluid which are forced radially inwards with respect to the axis of rotation 130 of the gantry 110 as a result of centrifugal forces acting on the fluid during the rotation of the component 120 around the axis of rotation. The bubbles are forced radially inwards because the bubbles have a density that is relatively lower than the density of the fluid. By removing the bubbles from the fluid in this manner, the impact of the bubbles on the performance of the X-ray tube, is reduced. Particles in the fluid in the reservoir 140 that have a density that is relatively lower than the density of the fluid would also be forced radially inwards, and likewise received in the chamber as a result of the centrifugal forces acting on the fluid. By removing such particles from the fluid in this manner, the impact of such particles on the performance of the X-ray tube, is also reduced.

During the rotation of a gantry of a CT imaging system, a component that is mechanically coupled to the gantry experiences a centrifugal acceleration. The centrifugal acceleration, $a$, can be calculated using the Equation:

$$a = (2\pi f)^2 \cdot r \qquad\qquad \text{Equation 1}$$

wherein $f$ is the frequency of rotation, and $r$ is the distance from the component to the axis of rotation. Using typical values for a conventional CT imaging scan, i.e. $f$ = 2.5 Hz, which corresponds to a rotation period of 400 milliseconds, and $r$ = 0.8 metres for the radius of a CT imaging system gantry, results in calculated value of $a$ = 197 $m/s^2$ for the centrifugal acceleration. This is approximately twenty times the earth's gravitational field strength. Under some operating conditions, the centrifugal acceleration may be even higher. In other words, under the typical operating conditions of a CT imaging system, the centrifugal force that is exerted on the bubbles in the reservoir is much greater than that due to gravity, and consequently it provides a significant improvement to the separation of the bubbles than is provided by gravity alone.

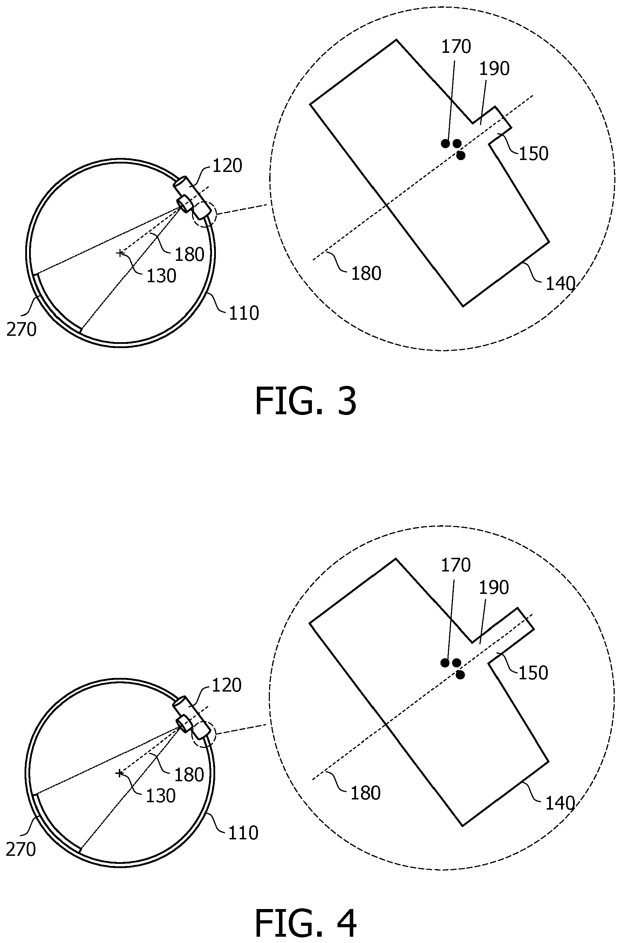

An alternative example that may be used to remove particles from the fluid in the reservoir instead of bubbles, is illustrated in Fig. 3. Fig. 3 is a schematic diagram illustrating an example of a component 120 that includes a reservoir 140, and a chamber 150 configured to receive particles 170, in accordance with some aspects of the present disclosure. Unless otherwise stated, items in Fig. 3 that have the same labels as those in Fig. 2 refer to the same item, and provide corresponding functionality. In the example illustrated in Fig. 3, the chamber 150 is fluidically coupled to the reservoir 140 via an opening 190 in the reservoir. The opening 190 is disposed at a radially outermost position of the reservoir 140 for receiving particles 170 in the fluid having a density that is greater than a density of the fluid and which are forced radially outwards with respect to the axis of rotation 130 of the gantry 110 as a result of centrifugal forces acting on the fluid during the rotation of the component 120 around the axis of rotation. Thus, the example illustrated in Fig. 3 differs from the example illustrated in Fig. 2, in that the opening 190 in the reservoir is disposed at a radially outermost position of the reservoir 140 for receiving particles 170 in the fluid. By removing the particles from the fluid in this manner, the impact of the particles on the performance of the X-ray tube, is reduced.

It is noted that the examples illustrated in Fig. 2 and Fig. 3 may also be combined by providing a first opening in the reservoir 140 in a radially innermost position, and a second opening in the reservoir in a radially outermost position. A first chamber that is fluidically coupled to the first opening may be used to receive bubbles from the fluid in the reservoir, and a second chamber that is fluidically coupled to the second opening may be used to receive particles from the fluid in the reservoir.

Various adaptations of the reservoirs illustrated in Fig. 2 and Fig. 3, are contemplated. These are described in the examples below. In general, these examples are described with reference to a reservoir 140 that includes a chamber 150 configured to receive particles 170, as in the example illustrated in Fig. 3. However, it is noted, that unless explicitly stated, these examples may alternatively be used with a reservoir 140 that includes a chamber 150 configured to receive bubbles 160, as in the example illustrated in Fig. 2.

In general, the particles or bubbles that are received by the chamber 150 are expected to have a size that is less than approximately 1 millimetre in diameter. Thus, in general, the opening 190 in the reservoir may have an aperture that is in the range from approximately 1 millimetre to 1 centimetre.

In general, it is noted that the reservoir 140 and the chamber 150 may have various shapes, and that the shape is not limited to the examples illustrated in the Figures. For instance, in the examples illustrated in the Figures, the inner surface of the reservoir 140 that includes the opening 190 is shaped in the form of a funnel for guiding the bubbles 160 or particles 170 into the chamber 150. This helps to prevent bubbles or particles from becoming stuck on the inner surface of the reservoir before entering the opening. However, it is to be appreciated that the surface of the reservoir 140 that includes the opening 190 may alternatively have a different shape to this example. In some examples, the inner surface of the reservoir 140 that includes the opening 190 includes one or more guiding structures, such as ribs, and which extend along the inner surface towards the opening 190 in order to guide the bubbles or particles towards the opening.

It is also noted that in the examples illustrated in the Figures, the chamber 150 is directly coupled to the reservoir 140. However, it is to be appreciated that the chamber 150 may alternatively be indirectly coupled to the reservoir 140. For instance, the reservoir 140 and the chamber may form part of a fluidic circuit, and the chamber may be 150 indirectly coupled to the reservoir 140 via the fluidic circuit. In one example, the reservoir 140 and the chamber 150 form part of a fluidic circuit that includes a heat exchanger and a pump for pumping the fluid around the fluidic circuit.

In one example, the chamber 150 comprises an elongate shape 200, and the elongate shape extends radially with respect to the axis of rotation 130 of the gantry. This example is illustrated in Fig. 4, which is a schematic diagram illustrating an example of a component 120 that includes a reservoir 140, and a chamber 150 with an elongate shape, in accordance with some aspects of the present disclosure. The example illustrated in Fig. 4 corresponds to the example illustrated in Fig. 3 with the exception that in that the example illustrated in Fig. 4, the chamber 150 has an elongate shape. Items in Fig. 4 that have the same labels as those in Fig. 3 refer to the same item, and provide corresponding functionality. In the example illustrated in Fig. 4, the elongate shape extends radially outwards with respect to the axis of rotation 130. The use of a chamber 150 with an elongate shape, i.e. a length-to-width ratio that is greater than unity, helps to improve the retention of particles 170 in the chamber 150 because particles that reach a distal end of the chamber need to travel a greater distance in order to escape back into the reservoir 140 than in a chamber that does not have an elongate shape. A chamber with an elongate shape may similarly be used to improve the trapping of bubbles in the example illustrated in Fig. 2. In this case, the elongate shape extends radially inwards with respect to the axis of rotation 130.

In another example, the chamber 150 comprises an elongate shape 200, and the chamber extends along a curved path in an opposing direction 210 to a direction of rotation 220 of the gantry, and the curved path forms at least a half-turn of a circle.

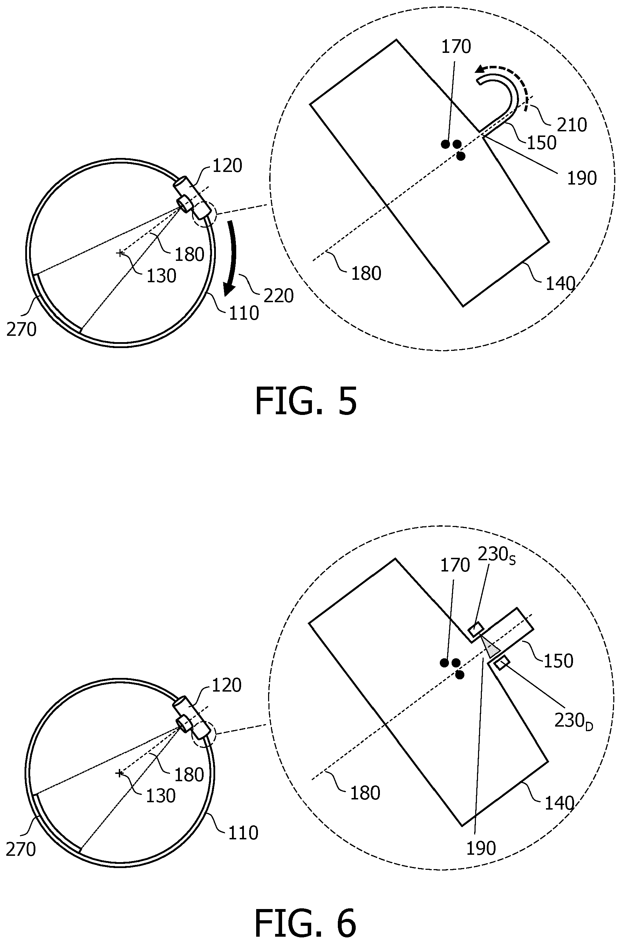

This example is illustrated in Fig. 5, which is a schematic diagram illustrating an example of a component 120 that includes a reservoir 140, and a chamber 150 with an elongate shape that extends along a curved path in an opposing direction 210 to a direction of rotation 220 of the gantry, in accordance with some aspects of the present disclosure. The example illustrated in Fig. 5 corresponds to the example illustrated in Fig. 4 with the exception that in that the example illustrated in Fig. 5, the chamber 150 extends along a curved path. Items in Fig. 5 that have the same labels as those in Fig. 4 refer to the same item, and provide corresponding functionality. The use of a chamber 150 with curved path in an opposing direction 210 to a direction of rotation 220 of the gantry, and which curved path forms at least a half-turn of a circle, further improves the retention of particles 170 in the chamber 150 because particles that are forced to a position in the chamber beyond a half-turn of a circle due to the centrifugal force generated by the rotation of the gantry, cannot escape back into the reservoir 140. A chamber with a similar shape may also be used to retain bubbles in the example illustrated in Fig. 2.

In a related example, the curved path comprises a spiral or a helix. With reference to the example illustrated in Fig. 5, the use of a chamber that extends along a curved path in the form of a spiral or helix, further improves the retention of particles in the chamber because as the particles are forced further along the spiral or helix path due to the centrifugal force generated by the rotation of the gantry (and similarly, for slow rotation also due to the gravitational force), the particles move progressively further from the opening 190, reducing the probability of the particles escaping back into the reservoir. The use of a curved path in the form of a spiral or a helix path also facilitates the provision of a long path with a compact form.

In another example, at least a portion of the chamber is detachable for removing bubbles or particles from the chamber. This facilitates maintenance to be performed on the fluid. By repetitively removing bubbles or particles via the detachable portion of the chamber and replacing it by a new detachable portion completely filled with clean fluid, the fluid in the reservoir 140 may be progressively cleaned.

In this example, the chamber may be detachable by providing the chamber with various fittings, such as a threaded fitting, a snap fitting, and a bayonet fitting, for example. In one example, the reservoir, and the detachable portion of the chamber, are provided with a self-locking valve. This helps to retain fluid within the reservoir during detachment of the detachable portion of the chamber.

In another example, a distal end of the chamber is provided with a valve for extracting the bubbles or particles. The bubbles or particles may be extracted from the chamber via the valve using a pump, for example.

In another example, the chamber includes a window for visually inspecting a contents of the chamber. The window may be provided by a glass, or a polymer, for example. The window facilitates a service engineer to determine whether there are particles or bubbles in the chamber, and consequently whether a maintenance operation should be performed on the fluid in the reservoir. In another example, bubbles, or particles are trapped or destroyed in the chamber. In this example, the chamber 150 comprises at least one of the following for trapping the bubbles or particles, or for destroying the bubbles: a plurality of electrodes configured to provide an electric field within the chamber, a semi-permeable membrane, a magnet, a filter, a catalyst, and an ultrasound transducer. By trapping or destroying the bubbles it is prevented that they can return to the reservoir.

Bubbles or particles are often electrically charged, and consequently they move under the influence of an electric field. In this examples, an electric field may be provided within the chamber by applying a DC potential difference across a pair of electrodes. The electric field generated between the electrodes causes bubbles or particles in the fluid to become trapped in the vicinity of an oppositely-charged electrode. The electrodes may be arranged in various positions within, or around the chamber 150. In one example, the electrodes are separated across an aperture of the chamber, such as across the opening 190, or alternatively deeper into the chamber along the radial direction. The electrodes trap the bubbles or particles as they are forced into the chamber under the centrifugal force generated by the rotation of the gantry.

A semi-permeable membrane permits the passage of specific molecules, gas, or small particles, via osmosis. In this example, the semi-permeable membrane is used to trap small particles that have passed through the semi-permeable membrane. The semi-permeable membrane may be arranged in various positions within the chamber 150. For example, the semi-permeable membrane may be provided across an aperture within the chamber, such as across the opening 190, or alternatively deeper into the chamber along the radial direction. Consequently, small particles that have passed through the semi-permeable membrane become trapped in the portion of the chamber beyond the semi-permeable membrane.

Some particles that appear in the fluid may be formed from ferromagnetic materials such as iron, steel, and so forth. Such particles may be generated as a result of mechanical wear in a pump in a fluidic circuit, for example. In this example, ferromagnetic particles are removed from the fluid by a (electro) magnet. The magnet may be arranged in various positions within or around the chamber 150 in order to provide a magnetic field within the chamber. In one example, the magnet is arranged in a wall of the chamber in order to trap ferromagnetic particles as they are forced into the chamber under the centrifugal force generated by the rotation of the gantry.

A filter may be used to trap particles, or trap or destroy bubbles. In this example, the filter may be arranged in various positions within the chamber 150. For example, the filter may be arranged across an aperture of the chamber, such as across the opening 190, or alternatively deeper into the chamber along the radial direction in order to trap or destroy bubbles or particles as they are forced into the chamber under the centrifugal force generated by the rotation of the gantry.

A catalyst may be used to trap particles, or dissolve or destroy bubbles.. The catalyst may be provided in the form of a hydrophilic (lipophobic) coating to an inner surface of the chamber 150, and which binds hydrophilic particles from fluids such as oil.

An ultrasound transducer may be used to deliver energy to the bubbles, causing them to burst. In this example, the ultrasound transducer may be disposed in various locations within the chamber, such as on wall of the chamber, for example, in order to destroy bubbles as they are forced into the chamber under the centrifugal force generated by the rotation of the gantry.

The wall may also be provided with a textured surface, or another geometry, that acts to trap particles or bubbles via surface tension.

In another example, the chamber 150 comprises at least one sensor $230_S$, $230_D$ for detecting a presence of bubbles 160 or particles 170 in the chamber.

This example is described with reference to Fig. 6, which is a schematic diagram illustrating an example of a component 120 that includes a reservoir 140, and a chamber 150 with a sensor $230_S$, $230_D$, in accordance with some aspects of the present disclosure. In this example, the sensor(s) may be provided by various types of sensor. For instance, the at least one sensor $230_S$, $230_D$ may include one or more of: an optical sensor, an impedance sensor, a chemical sensor, a thermal sensor, an acoustic sensor, an ultrasound sensor, and a mass sensor.

In the example illustrated in Fig. 6, an optical sensor is illustrated, and the optical sensor includes an optical source $230_S$, and an optical detector $230_D$. The optical source 230s and the optical detector $230_D$ are arranged to measure optical transmission in the chamber 190. The optical source 230s and the optical detector $230_D$ may be arranged at various positions within, or around, the chamber. For instance, the optical source $230_S$, and the optical detector $230_D$ may be arranged across an aperture of the chamber 150, such as across the opening 190, or alternatively deeper into the chamber along the radial direction in order to sense, via changes in the optical transmission, the passage of particles into the chamber as they are forced into the chamber under the centrifugal force generated by the rotation of the gantry. Various types of optical source may be used as the optical source $230_S$, such as a light emitting diode "LED", a laser, and a filament lamp, and so forth. Various types of optical detector may be used as the optical detector $230_D$, such as a photodiode, an avalanche photodiode, a photomultiplier tube, and so forth.

Particles in the fluid have the effect of increasing the optical scattering of the fluid. The scattering occurs due to the size of the particles in relation to the wavelength of optical radiation emitted by the optical source. The size of the particles is expected to be relatively smaller than a wavelength of the light emitted by the optical source, and consequently the particles are expected to scatter the optical radiation. Relatively larger particles may have the effect of interrupting the optical path between the optical source 230s and the optical detector $230_D$. In either case, in the example illustrated in Fig. 5, as particles accumulate in the fluid, the optical transmission of the fluid that is measured by the optical source 230s and the optical detector $230_D$, decreases. The optical sensor illustrated in Fig. 5 may be used to determine a total amount of particles that have accumulated in the chamber 190 by measuring the electrical signal that is generated by the optical detector $230_D$ in response to the detected radiation from the optical source $230_S$.

The optical sensor illustrated in Fig. 6 may be used in a similar manner in the example illustrated in Fig. 2 to detect bubbles. The bubbles are expected to interrupt the optical path between the optical source 230s and the optical detector $230_D$ because the bubbles are expected to be relatively larger than a wavelength of the light emitted by the optical source. In this case, as bubbles pass the optical sensor, the bubbles interrupt the radiation from the optical source $230_S$ that is detected by the optical detector $230_D$. This results in the generation of intermittent pulses in the electrical signal that is generated by the optical detector. Thus, an optical sensor may be used to determine a total amount of bubbles that have accumulated in the chamber 190 by counting, or integrating, the electrical pulses that are generated by the optical detector $230_D$. This information may be used in the prediction of future problems relating to the fluid. Bubbles that are relatively smaller than the wavelength of optical radiation emitted by the optical source have the effect of scattering optical radiation emitted by the optical source $230_S$, and consequently, as such bubbles accumulate in the fluid, the optical transmission of the fluid that is measured by the optical source $230_S$ and the optical detector $230_D$, may also decrease. Thus, the optical sensor may be used to determine a total amount of such bubbles that have accumulated in the chamber 190 by measuring the transmission of the optical path.

Other types of sensors may alternatively be used to detect bubbles or particles in the chamber in a similar manner. For instance, bubbles or particles in the fluid cause changes in the electrical impedance of the fluid, and consequently an impedance sensor may be used to detect a presence of the bubbles or particles. A chemical sensor may be used to detect the presence of specific chemicals in the fluid, and consequently to further classify the bubbles or particles as containing a specific gas, or molecule. A thermal sensor may be used to detect a presence of bubbles by measuring a thermal conductivity of the fluid, and which is affected by the amount of bubbles in the fluid. An acoustic sensor may be used to detect bubbles by means of intermittent changes in the sound, or acoustic impedance of the fluid as the bubbles are forced past the acoustic sensor into the chamber under the centrifugal force generated by the rotation of the gantry. The presence of bubbles in the chamber 190 also tends to reduce its total mass. Consequently, a mass sensor may be used to determine the mass of the chamber, and to thereby determine the presence of bubbles in the chamber via a reduction in its mass.

Thus, various types of sensor may be used to determine a total amount of bubbles or particles that have accumulated in the chamber 190. This may be useful in determining whether a maintenance operation should be performed on the fluid in the chamber.

In another example, the CT imaging system includes one or more processors 240, and the one or more processors are configured to control a rotation of the gantry 110. An example of a CT imaging system with one or more processors 240 is illustrated in Fig. 1. The one or more processors 240 may be used to provide various advantageous effects.

In some examples, the rotation of the gantry, as controlled by the one or more processors 240, is used to remove the bubbles or particles from the fluid, and thereby clean the fluid. In one example, the bubbles or particles are removed from the fluid in the reservoir during a conventional CT imaging scan. As described above, the centrifugal acceleration during a conventional CT imaging scan is approximately twenty or more times the earth's gravitational field strength, which provides a significant improvement to the separation of the bubbles that is provided by gravity alone. Thus, the fluid may be cleaned during routine use of the CT imaging system.

In another example, the bubbles or particles are removed from the fluid in the reservoir during a dedicated fluid-cleaning procedure. In the dedicated fluid-cleaning procedure, the gantry is rotated at a higher rotational frequency than is used for a conventional CT imaging scan, or for a longer duration than is used for a conventional CT imaging scan, in order to remove bubbles or particles from the fluid in the reservoir. As compared to a conventional CT imaging scan, this enhances the removal of bubbles or particles from the fluid in the reservoir.

In another example, the one or more processors 240 are configured to provide a parking position for the gantry. In this example the chamber 150 is fluidically coupled to the reservoir 140 via the opening 190 in the reservoir, and:

the opening 190 is disposed at a radially innermost position of the reservoir 140 for receiving bubbles 160 in the fluid which are forced radially inwards with respect to the axis of rotation 130 of the gantry 110 as a result of centrifugal forces acting on the fluid during the rotation of the component 120 around the axis of rotation; or
the opening 190 is disposed at a radially outermost position of the reservoir 140 for receiving particles 170 in the fluid having a density that is greater than a density of the fluid and which are forced radially outwards with respect to the axis of rotation 130 of the gantry 110 as a result of centrifugal forces acting on the fluid during the rotation of the component 120 around the axis of rotation.

When the gantry rotation slows down and is finally parked in the parking position, the gravitational force becomes dominant over the centrifugal force. Therefore, in this example, the parking position is defined by an angle of rotation $\phi$ of the gantry wherein:

the opening 190 disposed at the radially innermost position of the reservoir 140 is arranged uppermost with respect to the reservoir; or
the opening 190 disposed at the radially outermost position of the reservoir 140 is arranged lowermost with respect to the reservoir. This ensures that any trapped particles or bubbles remain trapped even without any gantry rotation.

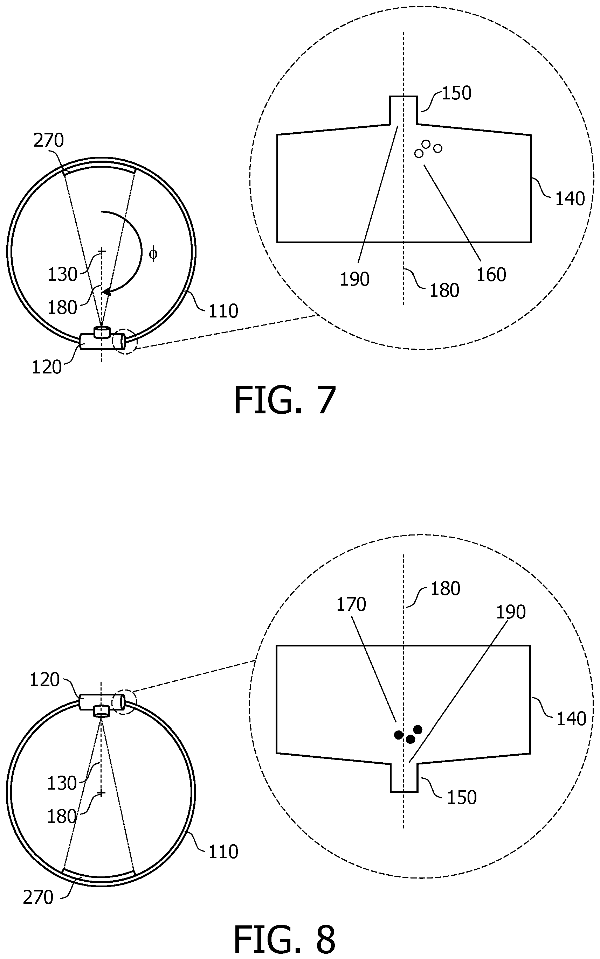

This example is described with reference to Fig. 7 and Fig. 8. Fig. 7 is a schematic diagram illustrating an example of a parking position for a gantry 110 wherein an opening 190 disposed at the radially innermost position of the reservoir 150 is arranged uppermost with respect to the reservoir, in accordance with some aspects of the present disclosure. The parking position illustrated in Fig. 7 prevents bubbles from escaping from the fluid in the chamber 150 when the CT imaging system is not in-use. The gantry may be arranged in the parking position by measuring the angle of rotation $\phi$ of the gantry, and stopping the gantry, or rotating the gantry, to the parking position via the one or more motors or actuators that control its rotation. The angle of rotation $\phi$ of the gantry in a CT imaging system is typically known from sensor measurements. The angle of rotation $\phi$ may be known from rotational sensor, or a position sensor. For example, a rotational encoder may be mechanically coupled to the one or more motors or actuators that control the rotation of the gantry and used to measure the angle of rotation $\phi$.

Fig. 8 is a schematic diagram illustrating an example of a parking position for a gantry 110 wherein an opening 190 disposed at the radially outermost position of the reservoir 150 is arranged lowermost with respect to the reservoir, in accordance with some aspects of the present disclosure. The parking position illustrated in Fig. 8 prevents particles from escaping from the fluid in the chamber 150 when the CT imaging system is not in-use. In the example illustrated in Fig. 8, the gantry may be arranged in the parking position in a similar manner to that described in relation to Fig. 7.

In another example, the one or more processors 240 are further configured to:

receive sensor data generated by the at least one sensor $230_S$, $230_D$;

determine a status of the chamber 150 based on the sensor data; and

transmit an indication of the chamber status to a remote processing device 250 via a data communication network 260 for triggering a service work order request to verify a functioning of the component 120, or to perform a maintenance operation on the component.

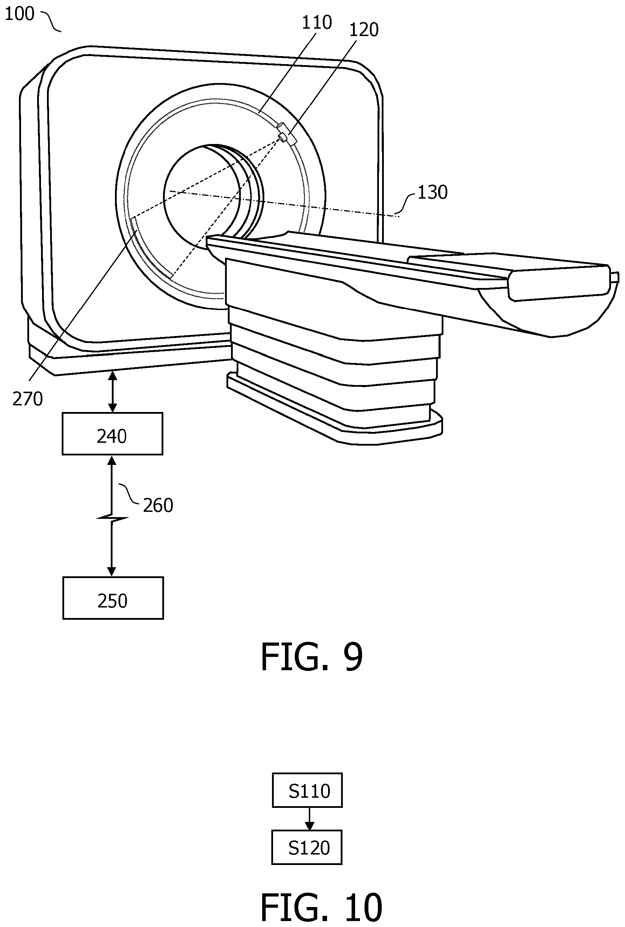

This example is described with reference to Fig. 9, which is a schematic diagram illustrating an example of a CT imaging system 100 including one or more processors 240 and a remote processing device 250, in accordance with some aspects of the present disclosure.

In this example, the status of the chamber represents a degree of filling of the chamber with bubbles or particles. The status of the chamber may be represented as an analogue or digital value. For instance, the status may be represented as "full" or "empty", or as a percentage of the filling. By triggering a service work order request to verify a functioning of the component 120, or to perform a maintenance operation on the component, it may be ensured that the performance of the component 120 is not degraded by the presence of bubbles or particles in the fluid. The indication of the chamber status may be transmitted via the data communication network 260 using any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

In another example, a component 120 for a CT imaging system 100, is provided. The component comprises:

a mounting for coupling the component to a rotatable gantry 110; and

wherein the mounting is configured such that when the component is mechanically coupled to the gantry 110, the gantry rotates the component 120 around an axis of rotation 130 of the gantry;

wherein the component 120 comprises a reservoir 140 for containing a fluid, and a chamber 150;

wherein the chamber 150 is fluidically coupled to the reservoir 140, and wherein the chamber is configured to receive bubbles 160 or particles 170 in the fluid which are forced radially 180 with respect to the axis of rotation 130 of the gantry 110 as a result of centrifugal forces acting on the fluid during the rotation of the component around the axis of rotation.

In this example, the component may be an X-ray tube, a generator, or an X-ray detector, for example. The mounting may be provided by various mechanical couplings that are suitable for coupling to a gantry of a CT imaging system. The mounting have a size and shape that is configured to couple with a corresponding coupling on the gantry. The mounting may include positions for one or more fastenings that are configured to mate with corresponding positions on the gantry. For instance, in the example of an X-ray tube, the X-ray tube may include a bracket with holes for receiving bolts, wherein the positions of the holes correspond to the positions of holes on a gantry of a CT imaging system.

In another example, a computer-implemented method of generating a service work order request for the component 120 of the CT imaging system 100 described with reference to Fig. 9, is provided. The method includes:

receiving S 110, at the remote processing device 250, the indication of the chamber status; and

automatically generating S 120 a service work order request to verify the functioning of the component 120, or to perform a maintenance operation on the component, based on the received indication.

This example is described with reference to Fig. 10, which is a flowchart illustrating an example of a computer-implemented method of generating a service work order request, in accordance with some aspects of the present disclosure.

In this example, the status of the chamber status represents a degree of filing of the chamber with bubbles or particles, as described above. By automatically triggering a service work order request, this example ensures that the performance of the component is maintained in an efficient manner.

The computer-implemented method may also be provided in the form of a computer program product. Thus, in another example, a computer program product for use with the component 120 of the CT imaging system 100 described with reference to Fig. 9, is provided. The computer program product comprises instructions which when executed by one or more processors, cause the one or more processors to carry out operations including:

receiving S 110, at the remote processing device 250, the indication of the chamber status; and

automatically generating S 120 a service work order request to verify the functioning of the component 120, or to perform a maintenance operation on the component, based on the received indication.

The above examples are to be understood as illustrative of the present disclosure, and not restrictive. Further examples are also contemplated. For instance, the examples described in relation to a CT imaging system, may also be provided by the component of the CT imaging system, in a corresponding manner. It is to be understood that a feature described in relation to any one example may be used alone, or in combination with other described features, and may be used in combination with one or more features of another of the examples, or a combination of other examples. Furthermore, modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

**Claims**

1.   A computed tomography, CT, imaging system (100) comprising:

   a rotatable gantry (110); and
   a component (120);
   wherein the component (120) is mechanically coupled to the gantry (110), and the gantry is configured to rotate the component around an axis of rotation (130);
   wherein the component comprises a reservoir (140) for containing a fluid, and **characterised by** a chamber (150);
   wherein the chamber (150) is fluidically coupled to the reservoir (140), and wherein the chamber is configured to receive bubbles (160) or particles (170) in the fluid which are forced radially (180) with respect to the axis of rotation (130) of the gantry (110) as a result of centrifugal forces acting on the fluid during the rotation of the component around the axis of rotation.

2.   The CT imaging system according to claim 1, wherein the chamber (150) is fluidically coupled to the reservoir (140) via an opening (190) in the reservoir, and wherein:

   the opening (190) is disposed at a radially innermost position of the reservoir (140) for receiving bubbles (160) in the fluid which are forced radially inwards with respect to the axis of rotation (130) of the gantry (110) as a result of centrifugal forces acting on the fluid during the rotation of the component (120) around the axis of rotation; or
   the opening (190) is disposed at a radially outermost position of the reservoir (140) for receiving particles (170) in the fluid having a density that is greater than a density of the fluid and which are forced radially outwards with respect to the axis of rotation (130) of the gantry (110) as a result of centrifugal forces acting on the fluid during the rotation of the component (120) around the axis of rotation.

3.   The CT imaging system according to claim 1, wherein the chamber (150) comprises an elongate shape (200), and wherein the elongate shape extends radially with respect to the axis of rotation (130) of the gantry.

4.   The CT imaging system according to claim 1, wherein the chamber (150) comprises an elongate shape (200), and wherein the chamber extends along a curved path in an opposing direction (210) to a direction of rotation (220) of the gantry, and wherein the curved path forms at least a half-turn of a circle.

5.   The CT imaging system according to claim 4, wherein the curved path comprises a spiral or a helix.

6.   The CT imaging system according to any previous claim, wherein at least a portion of the chamber is detachable for removing bubbles or particles from the chamber.

7.   The CT imaging system according to any previous claim, wherein the chamber (150) comprises at least one of the following for trapping the bubbles or particles, or for destroying the bubbles: a plurality of electrodes configured to provide an electric field within the chamber, a semi-permeable membrane, a magnet, a filter, a catalyst, and an ultrasound transducer.

8.   The CT imaging system according to any previous claim, wherein the chamber (150) comprises at least one sensor ($230_S$, $230_D$) for detecting a presence of bubbles (160) or particles (170) in the chamber.

9.   The CT imaging system according to claim 8, wherein the at least one sensor ($230_S$, $230_D$) comprises one or more of:

an optical sensor, an impedance sensor, a chemical sensor, a thermal sensor, an acoustic sensor, an ultrasound sensor, and a mass sensor.

10. The CT imaging system according to any previous claim, further comprising one or more processors (240); and wherein the one or more processors are configured to control a rotation of the gantry (110).

11. The CT imaging system according to claim 10 when dependent on claim 2, wherein the one or more processors (240) are further configured to provide a parking position for the gantry, and wherein the parking position is defined by an angle of rotation ($\phi$) of the gantry wherein:

the opening (190) disposed at the radially innermost position of the reservoir (140) is arranged uppermost with respect to the reservoir; or
the opening (190) disposed at the radially outermost position of the reservoir (140) is arranged lowermost with respect to the reservoir.

12. The CT imaging system according to claim 10 when dependent on claim 8, wherein the one or more processors (240) are further configured to:

receive sensor data generated by the at least one sensor ($230_S$, $230_D$);
determine a status of the chamber (150) based on the sensor data; and
transmit an indication of the chamber status to a remote processing device (250) via a data communication network (260) for triggering a service work order request to verify a functioning of the component (120), or to perform a maintenance operation on the component.

13. The CT imaging system according to any previous claim 1, wherein the component (120) comprises an X-ray tube, and wherein the fluid comprises a cooling fluid of the X-ray tube; or
wherein the component (120) comprises a voltage generator, and the fluid comprises an electrical isolation fluid and/ or cooling fluid of the generator.

14. A component (120) for a CT imaging system (100), the component comprising:

a mounting for coupling the component to a rotatable gantry (110); and
wherein the mounting is configured such that when the component is mechanically coupled to the gantry (110), the gantry rotates the component (120) around an axis of rotation (130) of the gantry;
wherein the component (120) comprises a reservoir (140) for containing a fluid, and **characterised by** a chamber (150);
wherein the chamber (150) is fluidically coupled to the reservoir (140), and wherein the chamber is configured to receive particles (170) in the fluid which are forced radially (180) with respect to the axis of rotation (130) of the gantry (110) as a result of centrifugal forces acting on the fluid during the rotation of the component around the axis of rotation.

15. A computer-implemented method of generating a service work order request for the component (120) of the CT imaging system (100) according to claim 12, the method comprising:

receiving (S110), at the remote processing device (250), the indication of the chamber status; and
automatically generating (S120) a service work order request to verify the functioning of the component (120), or to perform a maintenance operation on the component, based on the received indication.

**Patentansprüche**

1. Computertomographie-, CT-, Bildgebungssystem (100), umfassend:

ein drehbares Portal (110); und
eine Komponente (120);
wobei die Komponente (120) mechanisch mit dem Portal (110) gekoppelt ist und das Portal so konfiguriert ist, dass es die Komponente um eine Drehachse (130) dreht;
wobei die Komponente ein Reservoir (140) zum Enthalten eines Fluids umfasst und durch eine Kammer (150) gekennzeichnet ist;

wobei die Kammer (150) fluidisch mit dem Reservoir (140) gekoppelt ist, und wobei die Kammer so konfiguriert ist, dass sie Blasen (160) oder Partikel (170) in der Flüssigkeit empfängt, die infolge der während der Rotation der Komponente um die Drehachse auf die Flüssigkeit einwirkenden Zentrifugalkräfte in Bezug auf die Drehachse (130) des Portals (110) radial (180) gedrückt werden.

2. CT-Bildgebungssystem nach Anspruch 1, wobei die Kammer (150) über eine Öffnung (190) im Reservoir fluidisch mit dem Reservoir (140) gekoppelt ist, und wobei:

die Öffnung (190) an einer radial innersten Position des Reservoirs (140) angeordnet ist, um Blasen (160) in der Flüssigkeit zu empfangen, die infolge der während der Rotation der Komponente (120) um die Drehachse auf die Flüssigkeit einwirkenden Zentrifugalkräfte in Bezug auf die Drehachse (130) des Portals (110) radial nach innen gedrückt werden; oder

die Öffnung (190) an einer radial äußersten Position des Reservoirs (140) angeordnet ist, um Partikel (170) in dem Fluid zu empfangen, die eine Dichte aufweisen, die größer ist als eine Dichte des Fluids und die infolge der während der Rotation der Komponente (120) um die Drehachse auf das Fluid einwirkenden Zentrifugalkräfte in Bezug auf die Drehachse (130) des Portals (110) radial nach außen gedrückt werden.

3. CT-Bildgebungssystem nach Anspruch 1, wobei die Kammer (150) eine längliche Form (200) umfasst, und wobei sich die längliche Form radial in Bezug auf die Drehachse (130) des Portals erstreckt.

4. CT-Bildgebungssystem nach Anspruch 1, wobei die Kammer (150) eine längliche Form (200) umfasst, und wobei sich die Kammer entlang eines gekrümmten Pfads in einer entgegengesetzten Richtung (210) zu einer Drehrichtung (220) des Portals erstreckt und wobei der gekrümmte Pfad mindestens eine Halbkreiskurve bildet.

5. CT-Bildgebungssystem nach Anspruch 4, wobei der gekrümmte Pfad eine Spirale oder eine Helix umfasst.

6. CT-Bildgebungssystem nach einem der vorstehenden Ansprüche, wobei mindestens ein Abschnitt der Kammer abnehmbar ist, um Blasen oder Partikel aus der Kammer zu entfernen.

7. CT-Bildgebungssystem nach einem der vorstehenden Ansprüche, wobei die Kammer (150) zum Einfangen der Blasen oder Partikel oder zum Zerstören der Blasen mindestens eines von Folgendem umfasst: eine Vielzahl von Elektroden, die so konfiguriert sind, dass sie innerhalb der Kammer ein elektrisches Feld bereitstellen, eine semipermeable Membran, einen Magneten, einen Filter, einen Katalysator und einen Ultraschallwandler.

8. CT-Bildgebungssystem nach einem vorstehenden Anspruch, wobei die Kammer (150) mindestens einen Sensor ($_{230s,230D}$) zum Erkennen des Vorhandenseins von Blasen (160) oder Partikeln (170) in der Kammer umfasst.

9. CT-Bildgebungssystem nach Anspruch 8, wobei der mindestens eine Sensor ($_{230s,230D}$) eines oder mehreres umfasst von: einem optischen Sensor, einem Impedanzsensor, einem chemischen Sensor, einem thermischen Sensor, einem akustischen Sensor, einem Ultraschallsensor und einem Massensensor.

10. CT-Bildgebungssystem nach einem vorstehenden Anspruch, das weiter einen oder mehrere Prozessoren (240) umfasst; und

wobei der eine oder die mehreren Prozessoren so konfiguriert sind, dass sie eine Rotation des Portals (110) steuern.

11. CT-Bildgebungssystem nach Anspruch 10, sofern abhängig von Anspruch 2, wobei der eine oder die mehreren Prozessoren (240) weiter so konfiguriert sind, dass sie eine Parkposition für das Portal bereitstellen, und wobei die Parkposition durch einen Rotationswinkel ($\phi$) des Portals definiert ist, wobei:

die Öffnung (190), die an der radial innersten Position des Reservoirs (140) angeordnet ist, in Bezug auf das Reservoir ganz oben angeordnet ist; oder

die an der radial äußersten Position des Reservoirs (140) angeordnete Öffnung (190) in Bezug auf das Reservoir am untersten angeordnet ist.

12. CT-Bildgebungssystem nach Anspruch 10, sofern abhängig von Anspruch 8, wobei der eine oder die mehreren Prozessoren (240) weiter konfiguriert sind zum:

Empfangen von Sensordaten, die von dem mindestens einen Sensor (230$_s$, 230$_D$)erzeugt werden;

Bestimmen eines Zustands der Kammer (150) basierend auf den Sensordaten; und

Übertragen einer Angabe des Kammerzustands an eine entfernte Verarbeitungsvorrichtung (250) über ein Datenkommunikationsnetzwerk (260), um eine Service-Arbeitsauftragsanforderung auszulösen, um eine Funktion der Komponente (120) zu überprüfen oder einen Wartungsvorgang an der Komponente durchzuführen.

13. CT-Bildgebungssystem nach Anspruch 1, wobei die Komponente (120) eine Röntgenröhre umfasst und wobei das Fluid ein Kühlfluid der Röntgenröhre umfasst; oder

wobei die Komponente (120) einen Spannungsgenerator umfasst und das Fluid ein elektrisches Isolationsfluid und/oder ein Kühlfluid des Generators umfasst.

14. Komponente (120) für ein CT-Bildgebungssystem (100), wobei die Komponente Folgendes umfasst:

eine Halterung zum Koppeln der Komponente mit einem drehbaren Portal (110); und

wobei die Halterung so konfiguriert ist, dass, wenn die Komponente mechanisch mit dem Portal (110) gekoppelt ist, das Portal die Komponente (120) um eine Drehachse (130) des Portals dreht;

wobei die Komponente (120) ein Reservoir (140) zum Enthalten eines Fluids umfasst und durch eine Kammer (150) gekennzeichnet ist;

wobei die Kammer (150) fluidisch mit dem Reservoir (140) gekoppelt ist, und wobei die Kammer so konfiguriert ist, dass sie Partikel (170) in der Flüssigkeit empfängt, die infolge der während der Rotation der Komponente um die Drehachse auf die Flüssigkeit einwirkenden Zentrifugalkräfte in Bezug auf die Drehachse (130) des Portals (110) radial (180) gedrückt werden.

15. Computerimplementiertes Verfahren zum Erzeugen einer Service-Arbeitsauftragsanforderung für die Komponente (120) des CT-Bildgebungssystems (100) nach Anspruch 12, wobei das Verfahren Folgendes umfasst:

Empfangen (S110) der Angabe des Kammerzustands an der entfernten Verarbeitungsvorrichtung (250); und

automatisches Erzeugen (S120) einer Service-Arbeitsauftragsanforderung, um die Funktion der Komponente (120) zu überprüfen oder basierend auf der empfangenen Angabe einen Wartungsvorgang an der Komponente durchzuführen.

**Revendications**

1. Système d'imagerie par tomodensitométrie, TDM, (100) comprenant :

un portique rotatif (110) ; et

un composant (120) ;

dans lequel le composant (120) est couplé mécaniquement au portique (110), et le portique est configuré pour faire tourner le composant autour d'un axe de rotation (130) ;

dans lequel le composant comprend un réservoir (140) destiné à contenir un fluide, et **caractérisé par** une chambre (150) ;

dans lequel la chambre (150) est couplée fluidiquement au réservoir (140), et dans lequel la chambre est configurée pour recevoir des bulles (160) ou des particules (170) dans le fluide qui sont forcées radialement (180) par rapport à l'axe de rotation (130) du portique (110) sous l'effet de forces centrifuges agissant sur le fluide pendant la rotation du composant autour de l'axe de rotation.

2. Système d'imagerie TDM selon la revendication 1, dans lequel la chambre (150) est couplée fluidiquement au réservoir (140) via une ouverture (190) dans le réservoir, et dans lequel :

l'ouverture (190) est agencée dans une position radialement la plus intérieure du réservoir (140) pour recevoir des bulles (160) dans le fluide qui sont forcées radialement vers l'intérieur par rapport à l'axe de rotation (130) du portique (110) sous l'effet de forces centrifuges agissant sur le fluide pendant la rotation du composant (120) autour de l'axe de rotation ; ou

l'ouverture (190) est agencée dans une position radialement la plus extérieure du réservoir (140) pour recevoir des particules (170) dans le fluide présentant une densité qui est supérieure à une densité du fluide et qui sont forcées radialement vers l'extérieur par rapport à l'axe de rotation (130) du portique (110) sous l'effet de forces centrifuges agissant sur le fluide pendant la rotation du composant (120) autour de l'axe de rotation.

3. Système d'imagerie TDM selon la revendication 1, dans lequel la chambre (150) comprend une forme allongée (200), et dans lequel la forme allongée s'étend radialement par rapport à l'axe de rotation (130) du portique.

4. Système d'imagerie TDM selon la revendication 1, dans lequel la chambre (150) comprend une forme allongée (200), et dans lequel la chambre s'étend le long d'un trajet courbe dans une direction opposée (210) à une direction de rotation (220) du portique, et dans lequel le trajet courbe forme au moins un demi-tour d'un cercle.

5. Système d'imagerie TDM selon la revendication 4, dans lequel le trajet courbe comprend une spirale ou une hélice.

6. Système d'imagerie TDM selon l'une quelconque des revendications précédentes, dans lequel au moins une partie de la chambre est détachable pour éliminer les bulles ou les particules de la chambre.

7. Système d'imagerie TDM selon l'une quelconque des revendications précédentes, dans lequel la chambre (150) comprend au moins l'un des éléments suivants pour piéger les bulles ou les particules, ou pour détruire les bulles : une pluralité d'électrodes configurées pour fournir un champ électrique à l'intérieur de la chambre, une membrane semi-perméable, un aimant, un filtre, un catalyseur et un transducteur à ultrasons.

8. Système d'imagerie TDM selon l'une quelconque des revendications précédentes, dans lequel la chambre (150) comprend au moins un capteur ($230_s$, $230_D$) pour détecter une présence de bulles (160) ou de particules (170) dans la chambre.

9. Système d'imagerie TDM selon la revendication 8, dans lequel le ou les capteurs ($230_s$, $230_D$) comprennent un ou plusieurs éléments parmi : un capteur optique, un capteur d'impédance, un capteur chimique, un capteur thermique, un capteur acoustique, un capteur à ultrasons et un capteur de masse.

10. Système d'imagerie TDM selon l'une quelconque des revendications précédentes, comprenant en outre un ou plusieurs processeurs (240) ; et
dans lequel le ou les processeurs sont configurés pour commander une rotation du portique (110).

11. Système d'imagerie TDM selon la revendication 10, lorsqu'elle dépend de la revendication 2, dans lequel le ou les processeurs (240) sont en outre configurés pour fournir une position de stationnement pour le portique, et dans lequel la position de stationnement est définie par un angle de rotation ($\phi$) du portique dans lequel :

l'ouverture (190) disposée dans la position radialement la plus intérieure du réservoir (140) est agencée la plus haute par rapport au réservoir ; ou
l'ouverture (190) disposée dans la position radialement la plus extérieure du réservoir (140) est agencée la plus basse par rapport au réservoir.

12. Système d'imagerie TDM selon la revendication 10, lorsqu'elle dépend de la revendication 8, dans lequel le ou les processeurs (240) sont en outre configurés pour :

recevoir les données de capteur générées par le ou les capteurs ($230_s$, $230_D$) ;
déterminer un état de la chambre (150) sur la base des données de capteur ; et
transmettre une indication de l'état de la chambre à un dispositif de traitement à distance (250) via un réseau de communication de données (260) pour déclencher une demande de bon de travail d'entretien pour vérifier un fonctionnement du composant (120), ou pour effectuer une opération de maintenance sur le composant.

13. Système d'imagerie TDM selon la revendication 1, dans lequel le composant (120) comprend un tube à rayons X, et dans lequel le fluide comprend un fluide de refroidissement du tube à rayons X ; ou
dans lequel le composant (120) comprend un générateur de tension, et le fluide comprend un fluide d'isolation électrique et/ou un fluide de refroidissement du générateur.

14. Composant (120) pour un système d'imagerie TDM (100), le composant comprenant :

un support pour coupler le composant à un portique rotatif (110) ; et
dans lequel le support est configuré de telle sorte que, lorsque le composant est couplé mécaniquement au portique (110), le portique fasse tourner le composant (120) autour d'un axe de rotation (130) du portique ;

dans lequel le composant (120) comprend un réservoir (140) destiné à contenir un fluide, et **caractérisé par** une chambre (150) ;

dans lequel la chambre (150) est couplée fluidiquement au réservoir (140), et dans lequel la chambre est configurée pour recevoir des particules (170) dans le fluide qui sont forcées radialement (180) par rapport à l'axe de rotation (130) du portique (110) sous l'effet de forces centrifuges agissant sur le fluide pendant la rotation du composant autour de l'axe de rotation.

15. Procédé mis en œuvre par ordinateur pour générer une demande de bon de travail d'entretien pour le composant (120) du système d'imagerie TDM (100) selon la revendication 12, le procédé comprenant :

la réception (S110), au niveau du dispositif de traitement à distance (250), de l'indication de l'état de la chambre ; et

la génération automatique (S120) d'une demande de bon de travail d'entretien pour vérifier le fonctionnement du composant (120), ou pour effectuer une opération de maintenance sur le composant, sur la base de l'indication reçue.

FIG. 1

FIG. 2

FIG. 3

FIG. 4

**FIG. 5**

**FIG. 6**

FIG. 7

FIG. 8

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110243296 A1

- CN 112403038 A